Europäisches Patentamt

European Patent Office

Office européen des brevets

(19)

(11) **EP 0 311 648 B1**

(12) **EUROPÄISCHE PATENTSCHRIFT**

(45) Veröffentlichungstag und Bekanntmachung des Hinweises auf die Patenterteilung:
**31.01.1996  Patentblatt 1996/05**

(21) Anmeldenummer: **88902114.3**

(22) Anmeldetag: **10.03.1988**

(51) Int. Cl.$^6$: **A61N 1/00**, A61B 5/04,
A61N 1/30,  A61N 1/36

(86) Internationale Anmeldenummer: **PCT/CH88/00055**

(87) Internationale Veröffentlichungsnummer: **WO 88/07392 (06.10.1988  Gazette 1988/22)**

(54) **VERFAHREN ZUR SIGNALSTEUERUNG UND ANORDNUNG FÜR DIE ELEKTROBEHANDLUNG**

SIGNAL CONTROL PROCESS AND ELECTRIC TREATMENT DEVICE

PROCEDE DE REGULATION D'UN SIGNAL ET INSTALLATION POUR ELECTROTHERAPIE

(84) Benannte Vertragsstaaten:
**AT BE CH DE FR GB IT LI LU NL SE**

(30) Priorität: **26.03.1987 CH 1163/87**

(43) Veröffentlichungstag der Anmeldung:
**19.04.1989  Patentblatt 1989/16**

(60) Teilanmeldung: **95110865.3**

(73) Patentinhaber: **DYNAMIS Medizintechnik AG
CH-4143 Dornach (CH)**

(72) Erfinder: **MÜLLER, Felix
CH-8803 Rüschlikon (CH)**

(74) Vertreter: **Troesch, Jacques J., Dr. sc. nat. et al
CH-8050 Zürich (CH)**

(56) Entgegenhaltungen:
**EP-A- 0 029 245          WO-A-85/02124
WO-A-86/02567          DE-A- 2 658 096
DE-A- 3 207 050          DE-A- 3 236 756
DE-A- 3 318 874          DE-A- 3 618 748
FR-A- 1 360 384          FR-A- 2 191 824
US-A- 4 390 023**

**Beschreibung**

Die vorliegende Erfindung betrifft ein Verfahren zur Signalsteuerung bei der Elektrobehandlung von Lebewesen, wobei man mindestens zwei Stromkreise über das Lebewesen führt und eine Anordnung für die Elektrobehandlung von Lebewesen mit mindestens zwei Ausgängen zur Ausgabe elektrischer Reizsignale an eine Elektrodenanordnung für die Uebertragung der Reizsignale an das Lebewesen.

Für die Elektrobehandlung von Menschen oder Tieren ist es bekannt, letzteren, über Elektrodenanordnungen, elektrische Reizsignale auszuschalten. Dabei werden üblicherweise kontinuierliche Impulszüge aufgeschaltet, sei dies in Form von Rechteckimpulsen, Dreieckimpulsen, Trapezimpulsen oder von Sinus-Signalen.

Es ist im weiteren ebenfalls bekannt, gleichzeitig bis zu drei Stromkreise einem Menschen- oder Tierkörper aufzuschalten. Bereits bei drei, jedoch ausgesprochen bei mehr als drei Stromkreisen tritt folgendes Problem auf:

Der Körper stellt ein System verteilter Impedanzen dar. Jeder Körperpunkt ist mit jedem anderen elektrisch verbunden, so dass ein Strom, der am einen Ort einfliesst und an einem anderen Ort ausfliesst, sich innerhalb des Körpers nach den verteilten Impedanzverhältnissen seinen Weg sucht bzw. sich aufteilt. Dies führt beim gleichzeitigen Aufschalten von zwei, drei und insbesondere mehr derartige Stromkreise am Körper zu zunehmend nicht voraussagbaren, teilweise zeitvarianten, individuellen Reizüberlagerungen an Körperbereichen, indem sich die nicht voraussagbar durch den Körper fliessenden Stromanteile teils vektoriell addieren, teils subtrahieren. Dies führt beispielsweise zu nichtgewollten Ueberreizungen der einen Muskelpartien, während andere unterbelastet bleiben.

So zeigt beispielsweise DE 32 36 756 ein Reizstromtherapiegerät, welches mehrere voneinander unabhängige Stromkreise aufweist. Diese sollen aus den vorgehend genannten Gründen zeitlich gegeneinander versetzt mit reizwirksamen Strom beaufschlagt werden. Als reizwirksamer Strom wird ein Reizstromkurve resp. ein Impulszug bezeichnet, welcher aus mittelfrequenten Strömen aufgebaut ist. Diese Impulszüge dürfen sich nun nicht überschneiden. Nur ausnahmsweise dürfen sich zwei Impulszüge in sehr kurzen an- resp. abklingenden Phasen überlappen, weil dann die Reizüberlagerungen vernachlässigbar klein sein sollen.

Die vorliegende Erfindung setzt sich zur Aufgabe, ein Verfahren der genannten Art zu schaffen, mit dessen Hilfe zwei und mehr gleichzeitig aufgeschaltete und direkt reizwirksame Ströme sich bezüglich ihrer Reizwirkung nicht im Sinne einer Superposition beeinflussen.

Diese Aufgabe wird erfindungsgemäss durch die Merkmale nach Anspruch 1 gelöst.

Durch die erfindungsgemässe zeitliche Staffelung der individuellen Impulse, aus welchen die reizwirksamen Impulszüge aufgebaut werden, können mindestens zwei oder mehrere sich insgesamt überlappende Impulszüge gleichzeitig über das Lebewesen geführt werden, ohne dass eine Ueberlagerung oder Superposition der einzelenen Impulszüge untereinander auftritt. Im Gegensatz zu den bekannten Verfahren können somit vorteilhafterweise mehrere reizwirksame Impulszüge gleichzeitig eingesetzt werden.

Weiter wird von der Erkenntnis ausgegangen, dass die Reizung, wie von Muskelpartien, vornehmlich durch hochfrequente Signalanteile bewirkt wird. Somit wird weiter vorgeschlagen, dass Signalflanken als Impulssignale erzeugt werden, mit ihrem bekanntlich hohen Frequenzanteil.

Dadurch, dass man die Signalflanke mittels mindestens eines Impulses erzeugt, wird es auf einfache Art und Weise möglich, sie zeitlich zu stafffeln.

Unter Berücksichtigung, dass einerseits mit möglichst tiefen Signalpegeln eine möglichst hohe Reizwirkung erzielt werden soll, dass weiter generell dem Körper keine DC-Stromanteile aufgeschaltet werdensollten, wird weiter vorgeschlagen, dass man die Signalflanke zwischen zwei aufeinanderfolgenden Impulsen entgegengesetzter Polarität erzeugt.

In gewissen Fällen ist es angezeigt, eine Körperpartie rasch hintereinander mit mehreren Signalflanken zu reizen. In einem solchen Fall wird vorgeschlagen, mit dem Zeitabstand Signalflankenpakete zu erzeugen, wie Impulspakete. Es kann sich dabei um Sinusimpulspakete, Rechteckimpulspakete, Trapez- oder Dreieckimpulspakete handeln.

Im weiteren ist es in manchen Fällen erwünscht, eine zu behandelnde Körperpartie während der Behandlungszeit unterschiedlich zu reizen.

Damit nun eine behandelnde Person nicht manuell diesen Ablauf steuern muss und zudem eine exakte Reproduzierbarkeit gewährleistet ist, wird vorgeschlagen, dass man die Form und/oder die Stärke und/oder die Folgeperiode von Impulssignalen für mindestens einen der Stromkreise und/oder die Impulssignal-Zeitstaffelung für Stromkreise zwischen sich nach vorgebbaren zeitlichen Verläufen automatisch steuert.

Im weiteren stellt sich üblicherweise der im Körper fliessende Strom nach Massgabe der lokalen Impedanzverhältnisse aufgrund einer aufgeschalteten Reizsignalspannung ein. Als eigentliches Reizsignal ist aber der im Körper fliessende Strom massgebend. Somit kann der Fall auftreten, in welchem wegen der genannten lokalen Impedanzverhältnisse bei ein und derselben aufgeschalteten Reizsignalspannung an unterschiedlichen Körperteilen oder Individuen völlig unterschiedliche Reizströme fliessen.

Um dem Rechnung zu tragen, wird nun weiter vorgeschlagen, dass man einen Impulszug ausgangsseitig auf einen vorgebbaren SOLL-Verlauf regelt.

Eine Anordnung der obengenannten Art zeichnet sich zur Lösung der beschriebenen Aufgabe dadurch aus, dass erfindungsgemäss eine Generatoranordnung nach Anspruch 9 vorgesehen ist.

Eine einfache Realisationsform ergibt sich dadurch, dass die Generatoranordnung einen Generator zur Erzeugung einer Abfolge elektrischer Reizsignale umfasst sowie eine Zeitsteuereinheit zur zeitselektiven Ausgabe der Reizsignale auf die mindestens zwei Ausgänge.

Damit wird zentral die Reizsignalerzeugung vorgenommen, und mittels der dem Generator zugeordneten Zeitsteuereinheit werden die Reizsignale zeitselektiv den vorgesehenen, mindestens zwei Ausgängen aufgeschaltet.

In einfacher Art und Weise erfolgt dies weiter, indem die Generatoranordnung eine Multiplexer-Anordnung umfasst, um, wie erwähnt, zeitselektiv die Reizsignale auf die Ausgänge aufzuschalten.

Zur Festlegung der Zeitstaffelung der Impulssignale ist es wesentlich, am Generator zu definieren, welche Signalanteile als Impulssignale zu betrachten sind. Hierfür wird nun weiter vorgeschlagen, dass die Generatoranordnung als Impulssignal Signalflanken ausgibt. Somit ergibt sich die genannte Zeitstaffelung bezüglich dieser Signalflanken.

Um fallspezifisch die Intensität der Reizung einstellen zu können, wird weiter vorgeschlagen, dass Einstellorgane für die Flankenhöhe und/oder -Steilheit vorgesehen sind, wie verstärkungsgesteuerte Verstärker, Integratoren, Differentiatoren je mit unterschiedlichen oder variablen Zeitkonstanten.

Um im weiteren den Zeitmittelwert der Reizleistung wählen zu können, wird vorgeschlagen, dass die Generatoranordnung eine Einstellanordnung für die Folgefrequenz der Impulssignale umfasst und/oder für deren Zeitstaffelung.

Wie erwähnt, dauert eine Behandlung öfters längere Zeit. Während dieser Behandlungszeit wird die Intensität der Reizung je nach Behandlungszweck in der Zeit verstellt, z.B. während einer Aufwärmphase mit geringer Intensität, dann mit höherer und während einer Auslaufperiode wieder mit geringerer.

Um nun zu verhüten, dass Fachpersonal eine derartige Behandlung durch praktisch kontinuierliche Präsenz führen muss, wird weiter vorgeschlagen, dass die Anordnung eine Ablaufsteuereinheit umfasst sowie den Ausgängen zugeordnete Stellorgane für das elektrische Impulssignal, dass weiter die Ablaufsteuereinheit Speichermittel und Eingabeorgane für die Abspeicherung mindestens einer zeitlichen Abfolge von Stellsignalen umfasst und mit den Stellorganen verbunden ist, zum ausgangsspezifischen, automatischen Stellen der Impulssignale gemäss der zeitlichen Abfolge.

Des öftern ist es erwünscht, die Elektrobehandlung mit physiologischen Vorgängen der behandelten Person zu synchronisieren, wie beispielsweise mit Atmung, Herzrhythmus etc. Zu diesem Zweck wird weiter vorgeschlagen, dass die Ablaufsteuereinheit extern trigger oder synchronisierbar ist.

Während bei Vorsehen von bis zu drei Anordnungsausgängen die oben skizzierten Superpositionsprobleme manchmal noch voraussehbar sind und damit berücksichtigt werden können, so ergeben sich bei Vorsehen von mehr als drei Ausgängen - was oft erwünscht wäre - Verhältnisse, die sich der Voraussehbarkeit völlig entziehen. Aus diesem Grunde wird nun weiter vorgeschlagen, dass man an der erfindungsgemässen Anordnung mehr als drei Ausgänge vorsieht, da dies nun ohne weiteres möglich wird.

Bekanntlich ist es weiter aus Gründen der Elektrolyse der physiologischen Elektrolytlösungen zu vermeiden, einem menschlichen oder tierischen Körper einen DC-Strom aufzuschalten. Aus diesem Grunde wird weiter vorgeschlagen, dass die Ausgänge der Anordnung DC-entkoppelt werden, die je über einen transformatorischen Uebertrager.

Im weiteren wird vorgeschlagen, die Ausgänge potentialfrei zu betreiben, womit es der behandelnden Person freigestellt ist, festzulegen, welche Punkte auf gleichem Potential betrieben werden sollen, wenn nicht alle Stromkreise am Körper potentialfrei - fliegend - geschaltet werden sollen.

Um im weiteren von den lokalen Impedanzverhältnissen, Hautwiderstand, Schweissaussonderung etc. unabhängig zu werden, wird weiter vorgeschlagen, dass an mindestens einem Ausgang eine Strom- und/oder Spannungsmessvorrichtung vorgesehen ist, weiter eine diesem Ausgang zugeordnete Stellereinrichtung, die an einem Steuereingang mit einer Differenzeinheit verbunden ist, wobei einem der Eingänge der Differenzeinheit ein SOLL-Wert-Signal, einem zweiten der Ausgang der Messeinrichtung zugeführt ist.

Auf diese Art und Weise wird, unabhängig von den lokalen Impedanzverhältnissen, das dem Körper aufgeschaltete Impulszüge auf einen SOLL-Wert bzw. einen SOLL-Verlauf geregelt.

Die Erfindung wird nun beispielsweise anhand von Figuren erläutert.

Es zeigen:

Fig. 1       anhand eines Zeit-Impulssignaldiagrammes die erfindungsgemässe Zeitstaffelung von Impulssignalen für beispielsweise vier Ausgänge bzw. Reiz-Stromkreise,

Fig. 2       anhand von fünf Impulssignal-Zeitdiagrammen verschiedene Varianten erfindungsgemäss eingesetzter Impulszugverläufe,

Fig. 3a,b    anhand von Impulssignal-Zeitdiagrammen für vier gemeinsam betriebene Ausgänge, erfindungsgemäss ansteuerbare Impulszugabläufe,

Fig. 4       ein Signalfluss/Funktionsblock-Diagramm einer erfindungsgemässen Anordnung zur Erzeugung von Impulssignalen, wie der beispielsweise in den Fig. 1 - 3 gezeigten,

Fig. 5    anhand eines detaillierteren Funktionsblock-Diagrammes eine mögliche Ausbildung des Generators gemäss Fig. 4,

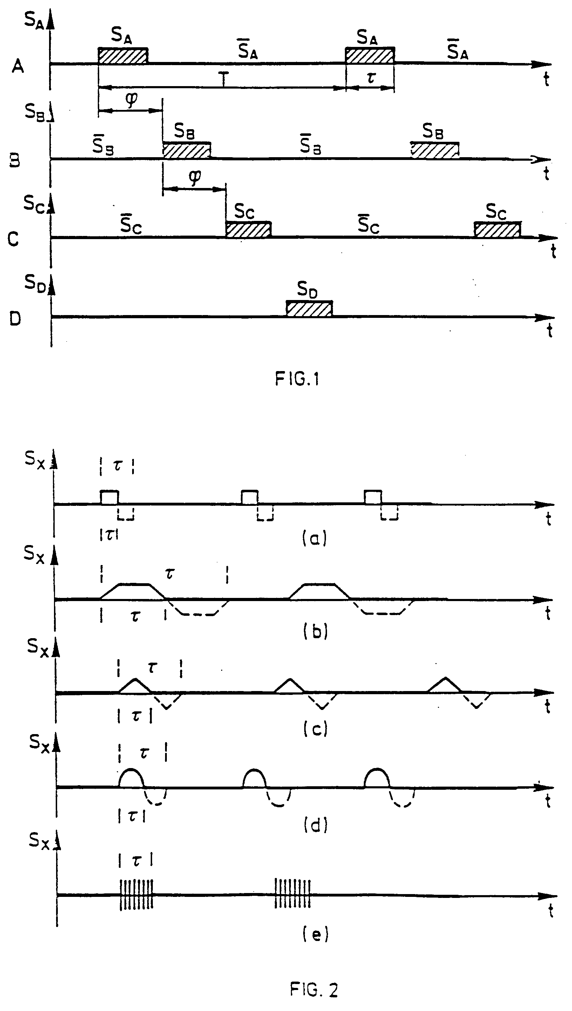

In Fig. 1 ist, anhand eines Zeitdiagrammes, für vier an den Körper eines Menschen oder Tieres bei einer Elektrobehandlung anzulegende Stromkreise, entsprechend Kanälen A bis C, die erfindungsgemässe Steuerung von Impulssignalen $S_A$ auf Kanal A und Impulssignalen $S_B$ auf Kanal B etc. dargestellt. Es handelt sich dabei vorzugsweise um Reiz-Spannungssignale, der Reizstrom stellt sich dann im Körper nach den Impedanzverhältnissen ein wie der Ausgangsstrom einer Spannungsquelle. Es kann sich aber auch um ein Reiz-Stromsignal handeln, dann stellt sich die Reizspannung nach Massgabe der genannten Impedanzverhältnisse ein wie bei der Ausgangsspannung einer Stromquelle. Wie ersichtlich, werden, bei jedem Kanal X (in Fig. 1 A bis C) einzeln betrachtet, Impulssignale $S_X$ oder Signale mit Reizsignalanteilen, während Zeitabschnitten $\tau$ ausgegeben. Diese Zeitabschnitte $\tau$, für jeden Kanal X betrachtet, folgen sich in regelmässigen oder unregelmässigen Zeitabständen T - $\tau$, wobei T die Folgeperiode der Impulssignalabschnitte $\tau$ bezeichnet. In den Zeitabschnitten T - $\tau$ treten somit, wie mit $\overline{S}_X$ dargestellt, keine Impulssignale auf. Erfindungsgemäss werden nun zwischen den vorgesehenen zwei oder mehr Kanälen X die Zeitabschnitte $\tau$ für oder mit Impulssignalen signalen derart in der Zeit verschoben, $\phi$, dass nie Impulssignale gleichzeitig auftreten, mindestens nicht in Kanälen, deren Ströme sich am Körper unerwünschterweise überlagern könnten: Dadurch wird erfindungsgemäss vermieden, dass sich an dem mit derartigen Impulssignalen beaufschlagten Körper unbeherrschbare Superpositionen von Strömen und damit Ueberreizungen einstellen können.

In Fig. 2 sind, ohne Anspruch auf Vollständigkeit, fünf verschiedene Arten von Impulssignalen a - e über der Zeit t dargestellt. Es kann sich dabei wie erwähnt um angelegte Spannungs- oder Stromsignale handeln.

Gemäss Fig. 2 kann es sich bei den Impulssignalen $S_X$ um monopolare oder vorzugsweise bipolare, gestrichelt dargestellt, Rechteckimpulse (a), Trapezimpulse (b), Dreieckimpulse (c), Sinusimpulse (d) oder um Impulspakete (e) beliebiger Einzelimpulsform handeln. Bevorzugterweise werden, wie gestrichelt dargestellt, Impulse bzw. Impulspakete so erzeugt, dass ihr linearer Zeitmittelwert verschwindet. Dies hat verschiedene Vorteile: Erstens wird dadurch sichergestellt, dass im zu behandelnden Körper kein DC-Strom fliesst. DC-Ströme bewirken in bekannter Weise Elektrolyse-Erscheinungen in körpereigenen Elektrolytlösungen, wie im Blut, mit den damit einhergehenden, schleichend gefährlichen Auswirkungen. Im übrigen ist es bekannt, dass hochfrequente Signalanteile besser in den Körper eindringen und eine intensivere Reizung ergeben. Somit sind insbesondere Signalflanken mit ihren hochfrequenten Signalanteilen als Impulssignale besonders wirksam. Werden nun, wie beschrieben, bipolare Impulse zur Erzeugung dieser Impulssignale beigezogen, so ergeben sich, verglichen mit den Flankenhöhen monopolarer Impulse, doppelte Flankenhöhen bei gleichbleibenden Impulshöhen. Somit wird das eigentliche Impulssignal beim Einsatz bipolarer Impulse mit Bezug auf den Einsatz monopolarer Impulse verdoppelt.

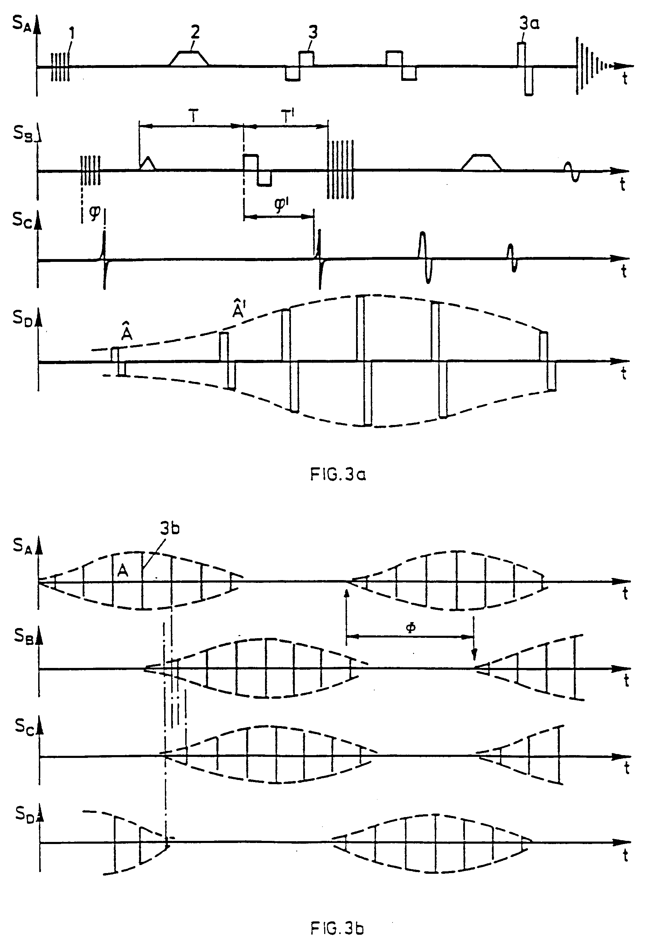

In Fig. 3a sind allgemeine Impulszüge für vier Stromkreise bzw. Kanäle A, B, C, D dargestellt. Es soll damit gezeigt werden, welche Steuerungs- bzw. Stellmöglichkeiten, einzeln betrachtet oder in Kombination, gemäss dem erfindungsgemässen Verfahren bzw. der entsprechenden Anordnung vorgesehen werden können. Wie bei Betrachtung der Signalverläufe an jedem Kanal A bis D einzeln ersichtlich, können in vorgebbarer zeitlicher Abfolge grundsätzlich einzelne oder mehrere der Impulssignal-Parameter geändert werden. So wird im Kanal A, wie dargestellt, die Impulssignalform von Impulspaket 1 zu monopolarem Trapezimpuls 2, bipolarem Rechteckimpuls 3 mit darauf folgender Erhöhung der Amplitude bei 3a und Verringerung der Impulsdauer und darauf zu einem in Amplitude abnehmendem Impulspaket gewechselt. Es können somit die Form der Impulssignale pro Kanal gestellt werden und/oder deren Höhe $\hat{A}$, $\hat{A}'$ und/oder die Folgefrequenz der Impulssignale entsprechend $\frac{1}{T}$, $\frac{1}{T'}$ und/oder die Zeitstaffelung $\phi$, $\phi'$ der Impulssignale zwischen den Kanälen.

In Fig. 3b sind analog zu Fig. 3a für vier Kanäle A bis D Impulssignaleverläufe $S_X$ dargestellt. In jedem der Kanäle werden Impulspakete mit bipolaren Einzelimpulsen 3b erzeugt, mit anund abschwellenden Amplitudenwerten A. Zwischen den Kanälen A bis D wird, wie strichpunktiert dargestellt, eine Zeitstaffelung entsprechend $\phi$ von Fig. 1 bzw. 3a eingehalten,und zwischen den Impulspaketen auf den Kanälen A bis D werden gesteuert einstellbare Zeitverschiebungen $\Phi$ eingehalten. Während in Fig. 3a ganz generell die Möglichkeiten aufgezeigt sind, wie die Impulssignale gesteuert werden können, ohne jeglichen Anspruch auf therapeutischen Sinn, sind in Fig. 3b diesbezüglich sinnvolle Signalverläufe dargestellt.

Die Impulssignal-Parameter können entweder manuell durch eine behandelnde Person nach vorgegebenem Plan verstellt werden oder, und bevorzugterweise, durch eine programmierbare Zeitablaufsteuerung, wie nachfolgend beschrieben werden wird.

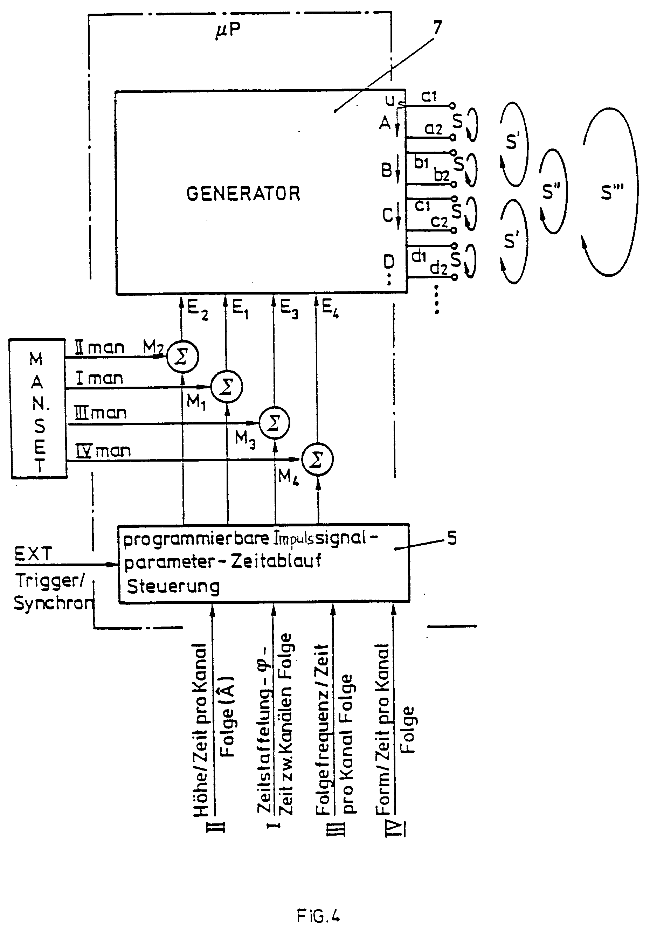

In Fig. 4 ist anhand eines Signalfluss/Funktionsblock-Diagrammes der prinzipielle Aufbau einer erfindungsgemässen Anordnung dargestellt, mit einer bevorzugterweise vorgesehenen Zeitablaufsteuerung 5. Die Anordnung umfasst eine Generatoreinheit 7 mit Ausgängen A, B ... entsprechend den bereits oben beschriebenen Kanälen. Die Impulssignale der oben beschriebenen Art werden an diesen Ausgängen erzeugt. Zur Schliessung von entsprechenden, mit den Pfeilen angedeuteten Reiz-Stromkreisen S, S', S", S'" über den Körper, umfassen die Ausgänge A, B etc. je zwei Anschlüsse, wobei zwischen diesen Anschlüssen erscheinende Spannungen $u_s$ von der Anordnung poten-

tialfrei erzeugt werden: Die Kanäle A, B, C, D etc. sind fliegend. Damit ist es einer zu behandelnden Person freigestellt, welche der einzelnen Ausgänge a1, a2 etc. zu ReizStromkreisen S, S' etc. kombiniert werden sollen.

In der einfachsten Ausführungsvariante steht die Generatoreinheit 7 allein und gibt an den Kanälen A, B, C, D mit fester Zeitverschiebung vorgegebene Impulssignale S aus. Bei einer ersten Ausführungsvariante ist ein erster Steuereingang $E_1$ vorgesehen, an welchem, zwischen den Kanälen, die Impulssignal-Zeitstaffelung entsprechend $\phi$ von Fig. 1 oder Fig. 3 eingestellt werden kann. Dabei kann entweder die Zeitstaffelung zwischen den Kanälen, wie zwischen A und B, B und C etc., synchron verstellbar sein, oder es kann, kanalspezifisch, die Zeitstaffelung an jedem Kanal individuell, wie bezüglich eines Referenzkanales, eingestellt werden. In einer weiteren Ausführungsvariante kann die Impulssignale-Amplitude $\hat{A}$ an einem Steuereingange $E_2$ für alle Kanäle gemeinsam oder kanalspezifisch verstellt werden. In einer weiteren Variante ist ein Steuereingang $E_3$ vorgesehen, an welchem die Impulssignal-Folgefrequenz entsprechend T, T' von Fig. 1 und Fig. 3 entweder für alle Kanäle gemeinsam oder wiederum kanalspezifisch verstellt werden kann. In einer noch weiteren Variante mit Steuereingang $E_4$ wird vorgesehen, an jedem Kanal die Impulssignal-Form wechseln zu können.

Zum Verstellen einzelner oder von Kombinationen der genannten Grössen an den Steuereingängen E ist gemäss Fig. 4 eine programmierbare Reizsignalparameter/Zeitablauf-Steuerung 5 vorgesehen. Sie umfasst Speicherorgane, wie programmierbare PROM oder EPROM, welche extern mit erwünschten Zeitabläufen für Impulssignal-Zeitstaffelung, Impulssignal-Amplitude, -Folgefrequenz, -Form geladen werden. Darnach steuert die Zeitablaufsteuerung 5 automatisch die Signalabfolge an den vorgesehenen Kanälen A, B, C, D etc., was beispielsweise in Verläufen gemäss Fig. 3a oder b resultiert. Gemäss Fig. 4 sind weiter manuell betätigbare Steuereingänge $M_1$ bis $M_4$ vorgesehen, womit alternativ zur automatischen Zeitablaufsteuerung auch manuell auf den genannten Zeitablauf eingegriffen werden kann. Die Zeitablaufsteuerung und die Generatoreinheit 7 mit den entsprechenden Steuerverbindungen werden bevorzugterweise durch Einsatz eines Mikroprozessors $\mu$P realisiert, wobei lediglich ausgangsseitig für die Kanäle A, B, C, D etc. eine digital-analoge Wandlung erfolgt.

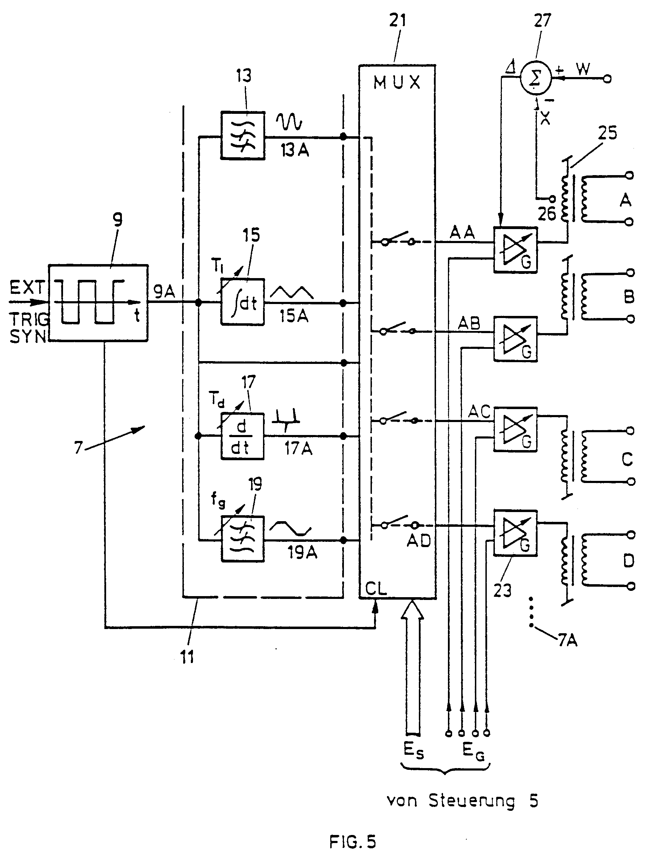

Ausgehend von der Darstellung gemäss Fig. 4 ist in Fig. 5 auf Basis von Funktionsblöcken eine einfache Realisationsvariante der Generatoreinheit 7 mit analogem Austrangsteil 7A dargestellt. Die Generatoreinheit 7 umfasst einen Rechteck-Impulsgenerator 9, der einen Rechteckimpulszug mit Tastverhältnis 50% abgibt. Der Ausgang $9_A$ des Impulsgenerators 9 wird einem Impulssignal-Formwandler 11 zugeführt. An einer Hochpassfiltereinheit 13 werden aus den Ausgangsimpulsen des Generators 9 an einem Ausgang $13_A$ sinusförmige Signale erzeugt. An einer Integrationseinheit 15, vorzugsweise mit einstellbarer Integrationszeitkonstanten Ti, werden aus den Rechteckimpulsen des Generator 9 Dreieckimpulse geformt. An einer Differentiations-Einheit 17, vorzugsweise mit einstellbarer Differentiationszeitkonstanten Td, werden kurze Impulse geformt. An einer Tiefpasseinheit 19, vorzugsweise mit einstellbarer Grenzfrequenz fg, werden Trapezsignale geformt. Die Ausgänge des Impulssignal-Formwandlers 11 werden einer Multiplex-Schaltung 21 zugeführt mit Kanalzugeordneten Ausgängen AA, AB, AC, AD. Jeder Eingang 13A, 15A etc. zur Multiplexer-Einheit 21 kann, wie gestrichelt für einen Eingang dargestellt, auf alle vorgesehenen, kanalspezifischen Ausgänge AX durchgeschaltet werden. Welcher Eingang zu welcher Zeit auf welchen kanalspezifischen Ausgang AX durchgeschaltet wird, wird über einen oder mehrere Steuereingänge $E_S$ zur Multiplexer-Einheit 21 angesteuert. Damit ermöglicht die bisher beschriebene Anordnung mit Generator 9, Impulssignal-Formwandler 11, Multiplexer-Einheit 21 die kanalspezifische Einstellung von Impulssignal-Form, -Folgefrequenz und die Einstellung der Zeitstaffelung $\phi$ zwischen dem Erscheinen der Impulssignale an den vorgesehenen Ausgängen AX. Zur Verstellung der Reizsignal-Höhe sind den Ausgängen AA, AB etc. der Multiplexer-Einheit 21 Verstärkereinheiten 23 mit verstellbarer Verstärkung G nachgeschaltet, wie beispielsweise Operationsverstärker mit kontinuierlich steuerbarem Gegenkopplungs-Netzwerk. Die Ausgänge dieser Verstärker 23 werden auf transformatorische Ausgangswandler 25 geführt, deren Sekundärwicklungen zur Bildung der Kanäle bzw. Ausgänge A, B, C, D fliegend, zum Anschliessen einer oder mehrerer entsprechender Elektrodenanordnungen, nach aussen geführt sind. Die Verstärkungen der Verstärkereinheiten 23 werden durch die Steuereingänge $E_G$ gestellt. Vorzugsweise erfolgt die Ansteuerung der Multiplexer-Schaltung 21 sowie der Verstärker 23 und allenfalls des Formwandlers 11 durch die Ablaufsteuerung 5 von Fig. 4.

Bezüglich des Kanals A ist in Fig. 5 eine zusätzliche Anordnung dargestellt. Dabei wird vorzugsweise primärseitig des transformatorischen Ausgangswandlers 25A der Reizstrom $i_s$ oder die Reizspannung $4_s$ bei 26 gemessen und als Regelgrösse X an einer Differenzeinheit 27 mit einer vorgebbaren entsprechenden Führungsgrösse W verglichen. Nach Massgabe der resultierenden Regeldifferenz $\Delta$ wird beispielsweise die Verstärkung G an der diesem Kanal A zugeordneten Verstärkereinheit 23 gestellt, derart, dass die Reizstromhöhe einem SOLL-Wert $\hat{i}_{SOLL}$ oder die Reizspannungshöhe einem SOLL-Wert $\hat{u}_{SOLL}$ entspricht. Selbstverständlich kann eine derartige regelnde Ueberwachung des Impulssignals S auch bezüglich anderer gesteuerter Impulssignal-Parameter erfolgen.

Die in hybrider Bauweise dargestellte Anordnung 7 wird bevorzugterweise digital realisiert, und es ergibt sich für den Fachmann aus den bisherigen Erläuterungen eine Grosszahl verschiedener Möglichkeiten, das erfindungsgemässe Verfahren bzw. die erfindungsgemässe Anordnung zu realisieren.

**Patentansprüche**

1. Verfahren zur Signalsteuerung von Impulszügen bei der Elektrobehandlung von Lebewesen, wobei man mindestens zwei Stromkreise (A,B) über das Lebewesen führt, dadurch gekennzeichnet, dass man zeitlich gestaffelte, sich nicht überschneidende individuelle Impulssignale (S) erzeugt und aus diesen Impulssignalen (S) in jeweils wenigstens zwei Stromkreisen (A,B) sich überlappende, sich wegen der zeitlichen Staffelung nicht beinflussende Impulszüge bildet.

2. Verfahren nach Anspruch 1, dadurch gekennzeichnet, dass man als Impulssignale (S) Signalflanken erzeugt, vorzugsweise mittels mindestens eines Impulses.

3. Verfahren nach Anspruch 2, dadurch gekennzeichnet, dass man die Signalflanke zwischen zwei aufeinanderfolgenden Impulsen entgegengesetzter Polarität erzeugt.

4. Verfahren nach einem der Ansprüche 2 bis 3, dadurch gekennzeichnet, dass man die Signalflanke durch mindestens einen Rechteck- (3,3a), Trapez- (2) oder Dreieckimpuls erzeugt.

5. Verfahren nach einem der Ansprüche 1 bis 4, dadurch gekennzeichnet, dass man mit dem Zeitabstand Signalflanken-Pakete (1) erzeugt, wie mittels eines Impulspaketes.

6. Verfahren nach einem der Ansprüche 1 bis 5, dadurch gekennzeichnet, dass man die Form und/oder Stärke und/oder die Folgeperiode (T) von Impulssignalen für mindestens einen der Stromkreise und/oder die Impulssignal-Zeitstaffelung ($\phi$) für Stromkreise zwischen sich nach vorgebbaren zeitlichen Verläufen automatisch steuert.

7. Verfahren nach einem der Ansprüche 1 bis 6, dadurch gekennzeichnet, dass man einen Impulszug auf einen vorgebbaren SOLL-Verlauf regelt.

8. Verfahren nach Anspruch 6, dadurch gekennzeichnet, dass man die Steuerung mit einem extern zugeführten Signal triggert oder synchronisiert.

9. Anordnung für die Elektrobehandlung von Lebewesen mit mindestens zwei Ausgängen zur Ausgabe von elektrischen Impulszügen an eine Elektrodenanordnung für die Uebertragung der Impulszüge an das Lebewesen, gekennzeichnet durch eine Generatoranordnung zur Erzeugung von zeitlich gestaffelten, sich nicht überschneidenden individuellen Impulssignalen (S) und zur Erzeugung an jeweils wenigstens zwei Ausgängen (A,B) von aus diesen Impulssignalen (S) gebildeten, sich überlappenden, wegen der zeitlichen Staffelung sich nicht beeinflussenden Impulszügen.

10. Anordnung nach Anspruch 9, dadurch gekennzeichnet, dass die Generatoranordnung einen Generator (9) zur Erzeugung einer Abfolge elektrischer Impulssignale umfasst sowie eine Zeitsteuereinheit (21) zur zeitselektiven Ausgabe der Reizsignale auf die mindestens zwei Ausgänge.

11. Anordnung nach Anspruch 9 oder 10, dadurch gekennzeichnet, dass die Generatoranordnung (9) als Impulssignal Signalflanken ausgibt.

12. Anordnung nach Anspruch 11, dadurch gekennzeichnet, dass Einstellorgane für die Flankenhöhe (23) und/oder -Steilheit (15, 17) vorgesehen sind.

13. Anordnung nach einem der Ansprüche 9 bis 12, dadurch gekennzeichnet, dass die Generatoranordnung eine Einstellanordnung (21) für die Folgefrequenz (T) der Reizsignale umfasst und/oder für die Zeitstafelung ($\phi$).

14. Anordnung nach einem der Ansprüche 9 bis 13, dadurch gekennzeichnet, dass die Generatoranordnung eine Multiplexer-Anordnung (21) umfasst, um, zeitselektiv, Impulssignale auf die Ausgänge durchzuschalten.

15. Anordnung nach einem der Ansprüche 9 bis 14, dadurch gekennzeichnet, dass die Generatoranordnung einen Rechteck- (9) und/oder Trapez- (13) und/oder Dreieck- (15) und/oder Sinus- (13) und/oder Impuls-Generator (17) umfasst.

16. Anordnung nach einem der Ansprüche 9 bis 15, dadurch gekennzeichnet, dass eine Ablaufsteuereinheit (5) vorgesehen ist, sowie den Ausgängen zugeordnete Stellorgane (21, 23) für das elektrische Impulssignal, dass weiter die Ablaufsteuereinheit (5) Speichermittel und Eingabeorgane für die Abspeicherung mindestens einer zeitlichen Abfolge von Stellsignalen umfasst und mit den Stellorganen (23, 21) verbunden ($E_S$, $E_G$) ist, zum ausgangsspezifischen automatischen Stellen der Impulssignale, gemäss der zeitlichen Abfolge, wobei vorzugsweise die Ablaufsteuereinheit auch extern trigger oder synchronisierbar ist.

17. Anordnung nach einem der Ansprüche 9 bis 16, dadurch gekennzeichnet, dass mehr als drei Ausgänge vorgesehen sind.

18. Anordnung nach einem der Ansprüche 9 bis 17, dadurch gekennzeichnet, dass die Ausgänge DC-entkoppelt sind (25).

19. Anordnung nach einem der Ansprüche 9 bis 18, dadurch gekennzeichnet, dass die Ausgänge potentialfrei geführt sind.

20. Anordnung nach einem der Ansprüche 9 bis 19, dadurch gekennzeichnet, dass an mindestens einem Ausgang (25A) eine Strom- und/oder Spannungsmessvorrichtung (26) vorgesehen ist, weiter eine diesem Ausgang zugeordnete Stellereinrichtung (21, 23), die an einem Steuereingang mit einer Differenzeinheit (27) verbunden ist, wobei einem der Eingänge der Differenzeinheit (27) ein SOLL-Wertsignal (W), einem zweiten der Ausgang der Messeinrichtung (26, X) zugeführt ist.

**Claims**

1. Method of signal control of pulse trains in the electrical treatment of living beings, wherein at least two electrical circuits (A, B) are passed through the living being, characterised in that individual pulse signals (S) arc generated which are chronologically staggered and do not cut across one another and from these pulse signals (S) in at least two electrical circuits (A, B) in each case overlapping pulse trains are formed which do not influence one another because of the chronological stagger.

2. Method as claimed in Claim 1, characterised in that signal edges are generated as pulse signals (S), preferably by means of at least one pulse.

3. Method as claimed in Claim 2, characterised in that the signal edge is generated between two successive pulses of opposing polarity.

4. Method as claimed in one of Claims 2 to 3, characterised in that the signal edge is generated by at least one rectangular (3, 3a), trapezoidal (2) or triangular pulse.

5. Method as claimed in one of Claims 1 to 4, characterised in that with the time interval signal edge sets are generated, as by means of a pulse set.

6. Method as claimed in one of Claims 1 to 5, characterised in that the shape and/or strength and/or the repetition period (T) of pulse signals for at least one of the electrical circuits and/or the chronological staggering of the pulse signals (φ) for electrical circuits between them is controlled automatically according to predeterminable chronological sequences.

7. Method as claimed in one of Claims 1 to 6, characterised in that a pulse train is adjusted to a predeterminable NOMINAL sequence.

8. Method as claimed in Claim 6, characterised in that the control is triggered or synchronised with an externally supplied signal.

9. Arrangement for the electrical treatment of living beings with at least two outputs for emitting electric pulse trains to an electrode arrangement for the transmission of the pulse trains to the living being, characterised by a generator arrangement for generating chronologically staggered individual pulse signals (S) which do not cut across one another and for generating at in each case at least two outputs (A, B) overlapping pulse trains which arc formed from these pulse signals (S) and do not influence one another because of the chronological stagger.

10. Arrangement as claimed in Claim 9, characterised in that the generator arrangement comprises a generator (9) for generating a succession of electrical pulse signals as well as a time control unit (21) for time-selective emission of the stimulating signals to the at least two outputs.

11. Arrangement as claimed in Claim 9 or 10, characterised in that the generator arrangement (9) emits signal edges as pulse signal.

12. Arrangement as claimed in Claim 11, characterised in that setting devices for the edge height (23) and/or the steepness of the edge (15, 17) are provided.

13. Arrangement as claimed in one of Claims 9 to 12, characterised in that the generator arrangement comprises a setting arrangement (21) for the repetition frequency (T) of the stimulating signals and/or for the chronological stagger (φ).

14. Arrangement as claimed in one of Claims 9 to 13, characterised in that the generator arrangement comprises a multiplexer arrangement (21) in order to switch pulse signals time-selectively through to the outputs.

15. Arrangement as claimed in one of Claims 9 to 14, characterised in that the generator arrangement comprises a rectangular (9) end/or trapezoidal (13) and/or triangular (15) and/or sinusoidal (13) and/or pulse generator (17).

16. Arrangement as claimed in one of Claims 9 to 15, characterised in that a sequence control unit (5) is provided, as well as setting devices (21, 23) co-ordinated with the outputs for the electrical pulse signal, and that the sequence control unit (5) comprises storage means and input device, for the storage of at least one chronological succession of setting signals and is connected ($E_S$, $E_G$) to the setting devices (23, 21), for output-specific automatic setting of the pulse signals, according to the chronological succession, wherein preferably the sequence control unit con also be externally triggered or synchronised.

17. Arrangement as claimed in one of Claims 9 to 16, characterised in that more than three outputs are provided.

18. Arrangement as claimed in one of Claims 9 to 17, characterised in that the outputs are DC-decoupled (25).

19. Arrangement as claimed in one of Claims 9 to 18, characterised in that the outputs are operated without potential.

20. Arrangement as claimed in one of Claims 9 to 19, characterised in that a current and/or voltage measuring device (26) is provided at at least one output (25A), and also a setting arrangement (21, 23) coordinated with this output and connected to a control input with a differential unit (27), wherein a NOMINAL value signal (W) is supplied to one of the inputs of the differential unit (27) and the output of the measuring arrangement (26, X) is supplied to a second input.

**Revendications**

1. Procédé pour la commande de signaux de trains d'impulsions lors du traitement par électrothérapie d'êtres vivants, selon lequel on fait passer au moins deux circuits (A, B) sur l'être vivant, caractérisé en ce qu'on génère des signaux d'impulsions individuels (S) échelonnés dans le temps et qui ne se coupent pas, et à partir des signaux d'impulsions (S) se forment dans au moins deux circuits (A, B) des trains d'impulsions qui se recouvrent et qui ne s'influencent pas en raison de l'échelonnement dans le temps.

2. Procédé selon la revendication 1, caractérisé en ce qu'on génère comme signaux d'impulsions (S) des flancs de signaux, de préférence à l'aide d'au moins une impulsion.

3. Procédé selon la revendication 2, caractérisé en ce qu'on génère le flanc de signal entre deux impulsions successives de polarités opposées.

4. Procédé selon l'une des revendications 2 à 3, caractérisé en ce qu'on génère le flanc de signal grâce à au moins une impulsion carrée (3, 3a), trapézoïdale (2) ou triangulaire.

5. Procédé selon l'une des revendications 1 à 4, caractérisé en ce qu'on génère suivant un intervalle de temps des paquets de flancs de signaux (1), par exemple à l'aide d'un paquet d'impulsions.

6. Procédé selon l'une des revendications 1 à 5, caractérisé en ce qu'on commande automatiquement la forme et/ou l'intensité et/ou la période de répétition (T) de signaux d'impulsions pour l'un au moins des circuits et/ou l'échelonnement dans le temps (φ) des signaux d'impulsions pour les circuits entre eux en fonction de variations dans le temps aptes à être prédéfinies.

7. Procédé selon l'une des revendications 1 à 6, caractérisé en ce qu'on règle un train d'impulsions sur une courbe de consigne apte à être prédéfinie.

8. Procédé selon la revendication 6, caractérisé en ce qu'on déclenche ou on synchronise la commande avec un signal transmis de l'extérieur.

9. Dispositif pour le traitement par électrothérapie d'êtres vivants, comportant au moins deux sorties destinées à l'émission de trains d'impulsions électriques au niveau d'un dispositif d'électrodes prévu pour transmettre les trains d'impulsions à l'être vivant, caractérisé par un dispositif générateur destiné à générer des signaux d'impulsions individuels (S) échelonnés dans le temps et qui ne se coupent pas, et à générer au niveau d'au moins deux sorties (A, B) des trains d'impulsions qui sont formés à partir de ces signaux d'impulsions (S), qui se recouvrent et qui ne s'influencent pas en raison de l'échelonnement dans le temps.

10. Dispositif selon la revendication 9, caractérisé en ce que le dispositif générateur comprend un générateur (9) destiné à générer une succession de signaux d'impulsions électriques, et une unité de commande dans le temps (21) destinée à émettre de façon sélective dans le temps les signaux d'excitation aux deux sorties ou plus.

11. Dispositif selon les revendications 9 ou 10, caractérisé en ce que le dispositif générateur (9) émet, comme signal d'impulsion, des flancs d'impulsion.

12. Dispositif selon la revendication 11, caractérisé en ce qu'il est prévu des organes de réglage pour la hauteur (23) et/ou la pente (15, 17) des flancs.

13. Dispositif selon l'une des revendications 9 à 12, caractérisé en ce que le dispositif générateur comprend un dispositif de réglage (21) pour la fréquence de répétition (T) des signaux d'excitation et/ou pour l'echelonnement dans le temps (φ).

14. Dispositif selon l'une des revendications 9 à 13, caractérisé en ce que le dispositif générateur comprend un dispositif multiplexeur (21) afin de transmettre de façon sélective dans le temps des signaux d'impulsions aux sorties.

15. Dispositif selon l'une des revendications 9 à 14, caractérisé en ce que le dispositif générateur comprend un générateur d'impulsions carrées (9) et/ou un générateur d'impulsions trapézoïdales (19) et/ou un générateur d'impulsions triangulaires (15) et/ou un générateur d'impulsions sinusoïdales (13) et/ou un générateur d'impulsions (17).

16. Dispositif selon l'une des revendications 9 à 15, caractérisé en ce qu'il est prévu une unité de commande de déroulement (5) et des organes de réglage (21, 23) associés aux sorties pour le signal d'impulsion électrique, et en ce que l'unité de commande de déroulement (5) comprend des moyens de mise en mémoire et des organes d'entrée pour la mise en mémoire d'au moins une succession dans le temps de signaux de réglage, et est reliée ($E_S$, $E_G$) aux organes de réglage (23, 21) en vue du réglage automatique, spécifique à la sortie, des signaux d'impulsion selon la succession dans le temps, l'unité de commande de déroulement étant de préférence apte à être déclenchée ou synchronisée également de l'extérieur.

17. Dispositif selon l'une des revendications 9 à 16, caractérisé en ce qu'il est prévu plus de trois sorties.

18. Dispositif selon l'une des revendications 9 à 17, caractérisé en ce que les sorties sont découplées (25) par rapport au courant continu.

19. Dispositif selon l'une des revendications 9 à 18, caractérisé en ce qu'on fait fonctionner les sorties sans potentiel.

20. Dispositif selon l'une des revendications 9 à 19, caractérisé en ce qu'il est prévu, à au moins une sortie (25A), un dispositif ampèremètre et/ou voltmètre (26) et, associé à cette sortie, un dispositif de réglage (21, 23) qui est relié, au niveau d'une entrée de commande, à une unité différenciatrice (27), étant précisé qu'un signal de valeur de consigne (W) est transmis à l'une des entrées de cette unité (27) tandis que la sortie du dispositif de mesure (26, X) est reliée à une seconde entrée.

FIG.1

FIG. 2

FIG.3a

FIG.3b

FIG.4

FIG. 5

von Steuerung 5